# FASCICULE DE BREVET EUROPEEN

(11) **EP 1 903 101 B1**
(45) Date de publication et mention de la délivrance du brevet: **11.11.2009**
(21) Numéro de dépôt: 07291108.4
(22) Date de dépôt: 17.09.2007
(51) Int. Cl.: C12M 1/00, C12M 1/26, C12M 3/00

(54) **Procédé de renouvellement d'un milieu biologique dans lequel des cellules sont en suspension**
Verfahren zur Erneuerung eines biologischen Mediums, in dem Zellen in Suspension sind
Method of renewing a biological environment containing cells in suspension

(30) Priorité: 22.09.2006 FR 0608347
(43) Date de publication de la demande: 26.03.2008
(73) Titulaire: MACO PHARMA, 59420 Mouvaux (FR)
(72) Inventeur: Heron, Antoine, 59250 Halluin (FR); Coissac Blondel, Cécile, 59000 Lille (FR)
(74) Mandataire: Sayettat, Julien Christian

(56) Documents cités:
- WO-A-01/53451
- WO-A-87/06952

## Description

L'invention concerne un procédé de renouvellement d'un milieu biologique ainsi qu'un système de transfert pour la mise en oeuvre d'un tel procédé et un ensemble de culture cellulaire comprenant un tel système de transfert.

L'invention s'applique notamment dans le cas où des cellules en suspension dans un milieu de culture, notamment un milieu nutritif, doivent être mises en suspension dans un nouveau milieu de culture.

La culture ou la prolifération de cellules est réalisée en mettant en contact des cellules avec un milieu nutritif, qui doit être renouvelé périodiquement pour assurer le bon approvisionnement des cellules en éléments nutritifs et extraire les déchets métaboliques et produits sécrétés dans le milieu qui sont potentiellement toxiques.

Cette étape de renouvellement ou changement de milieu de culture est réalisée en système ouvert, c'est-à-dire en mettant la suspension de cellules en contact avec l'air ambiant, créant ainsi un risque de contamination des cellules.

Par exemple, lorsque les cellules sont cultivées dans des récipients rigides tels que des flacons ou des boîtes de Petri, les cellules sont prélevées des récipients rigides à l'aide d'une pipette et transférées dans des tubes qui sont ensuite centrifugés. Les cellules centrifugées sont alors remises en suspension dans un nouveau milieu de culture et replacées dans le récipient rigide par simple déversement ou à l'aide d'une pipette.

Cette étape de centrifugation, en plus d'ouvrir le système de culture, fragilise les cellules.

On connaît du document W08706952 un système pour réaliser l'introduction d'un milieu de culture dans une poche de culture. Mais ce système ne permet pas de réaliser, pour des cellules en suspension, le changement de milieu de culture ou la concentration de cellules, c'est-à-dire le retrait d'au moins une partie du milieu nutritif de la suspension cellulaire.

L'invention propose donc un procédé sécurisé de renouvellement d'un milieu biologique dans lequel des cellules sont en suspension, permettant de s'assurer qu'un minimum de cellules soit perdues ou endommagées pendant ce renouvellement.

A cet effet, et selon un premier aspect, l'invention propose un procédé de renouvellement d'un milieu biologique dans lequel des cellules sont en suspension, ledit procédé prévoyant de :
- mettre en communication la suspension avec une source de milieu biologique de renouvellement par l'intermédiaire d'une voie de communication, ladite voie comprenant un moyen de rétention des cellules et, entre le moyen de rétention et la source, un moyen d'évacuation du milieu biologique ;
- évacuer dans le moyen d'évacuation le milieu biologique de la suspension par passage au travers du moyen de rétention dans un premier sens ; puis
- transférer le milieu de renouvellement au travers du moyen de rétention dans un deuxième sens, pour mettre en suspension les cellules avec le milieu de renouvellement, ledit deuxième sens étant prévu pour renvoyer dans la suspension les éventuelles cellules retenues dans le dispositif de rétention.

Selon un deuxième aspect, l'invention propose un système de transfert pour la mise en oeuvre du procédé selon le premier aspect, ledit système comprenant une première tubulure dont l'une des extrémités est pourvue d'un moyen de mise en communication avec le récipient contenant la suspension et l'autre extrémité est pourvue d'un moyen de mise en communication avec le container contenant le milieu biologique de renouvellement, un moyen de rétention étant connecté sur ladite tubulure, une deuxième tubulure étant connectée à la première tubulure entre le moyen de rétention et l'extrémité la deuxième extrémité, un container de recueil étant branché sur la deuxième tubulure.

Selon un troisième aspect, l'invention concerne un ensemble de culture cellulaire comprenant un système de transfert selon le deuxième aspect de l'invention, comprenant :
- un récipient de culture dans lequel sont placés des cellules et un premier milieu biologique comprenant au moins une voie d'accès, ladite voie étant connectée à l'une des extrémités de la première tubulure ;
- un container dans lequel est placé un milieu biologique de renouvellement et comprenant au moins un orifice d'entrée/sortie, ledit orifice étant connecté à l'autre extrémité de la première tubulure.

D'autres objets et avantages apparaîtront au cours de la description qui suit en référence aux dessins annexés.

La figure 1 représente de façon schématique un récipient de culture destiné à être associé à un système de transfert selon l'invention.

La figure 2 représente de façon schématique un système de transfert selon une première variante de l'invention.

La figure 3 représente de façon schématique un système de transfert selon une deuxième variante de l'invention.

La figure 4 représente de façon schématique un ensemble pour culture cellulaire comprenant le système de transfert de la figure 2.

La culture cellulaire désigne un ensemble de techniques utilisées pour faire croître des cellules hors de leur organisme ("ex-vivo") ou de leur milieu d'origine, dans un but thérapeutique, d'expérimentation scientifique ou de production biotechnologique.

Parmi ces techniques, l'ensemencement comprend la mise en culture de cellules dans un récipient de culture. Cet ensemencement est réalisé en mettant en suspension des cellules dans un milieu de culture.

Le renourrissage consiste en un changement ou renouvellement du milieu de culture. Dans ce cas, le milieu de culture est prélevé du récipient et remplacé par un milieu de culture frais. Cette technique est utilisée pour apporter aux cellules en culture les éléments nécessaires à leur maintien, prolifération, expansion et/ou différentiation.

A la fin de la culture, les cellules sont repiquées, c'est-à-dire qu'elles sont concentrées et retirées du récipient de culture pour être récupérées ou réensemencées dans d'autres récipients de culture.

En variante, à la fin de la culture, le surnageant sans cellules pouvant contenir des produits d'intérêt est récupéré.

Selon un premier aspect, l'invention fournit un procédé de renouvellement d'un milieu biologique dans lequel des cellules sont en suspension, évitant la perte et l'endommagement des cellules lors des manipulations.

Les cellules sont par exemple des cellules animales, notamment humaines, destinées à être injectées à un patient dans un but thérapeutique. Les cellules sont en particulier des cellules du type non-adhérentes.

Le milieu biologique est notamment un milieu adapté à l'expansion ou le maintien des cellules, par exemple un milieu nutritif. Le milieu nutritif peut être un milieu de base disponible dans le commerce, tel que Dulbecco's Modified Eagle's Medium (DMEM), Minimal Essential Medium (MEM), CMRL, EMEM, RPMI1640, DMEM/F-10, DMEM/F12.

Ce milieu de base peut être supplémenté avec divers additifs couramment utilisés par l'homme du métier. Parmi les additifs, on peut citer du sérum ou un substitut de sérum, des acides aminés non essentiels, la L-glutamine, des vitamines, des antibiotiques, le béta-mercaptoéthanol, des facteurs de croissance, des cytokines, etc.

Selon une variante, le milieu biologique est un milieu de différentiation permettant, par exemple, la différentiation de cellules souches en cellules spécifiques.

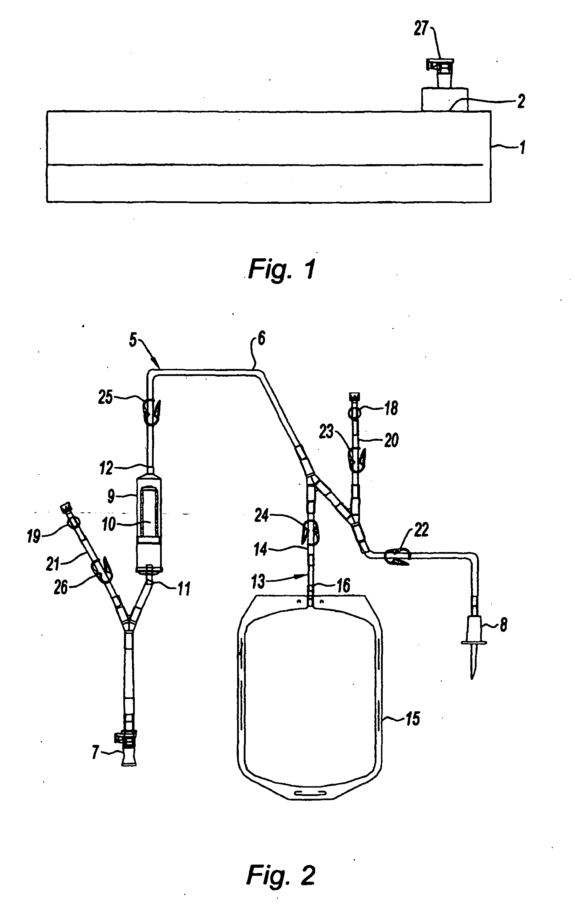

Dans une réalisation particulière, les cellules en suspension dans le milieu biologique sont placées dans un récipient 1 adapté à la culture de cellules tel que celui représenté sur la figure 1.

Un tel récipient 1 comprend au moins une voie d'accès 2 pour l'introduction et/ou le retrait des cellules et des milieux biologiques dans le récipient 1.

Le récipient a des propriétés de perméabilité aux gaz et de non-adhérence aux cellules adaptées aux différents types de cellules à cultiver.

Le récipient est notamment un flacon rigide et transparent, réalisé par exemple en un matériau polymère biocompatible tel que le polystyrène.

D'autres matériaux biocompatibles pour réaliser le récipient sont le polypropylène, l'éthylène-acétate de vinyl, le Teflon® ou le polycarbonate.

En variante, le récipient peut être un récipient à plusieurs étages, tel que celui décrit dans l'US6569675.

Selon une réalisation, la voie d'accès 2 du récipient est fermée par un bouchon amovible, muni d'une membrane poreuse perméable aux gaz et imperméable aux liquides. Cette membrane permet les échanges gazeux nécessaires à la culture des cellules dans le récipient de culture.

Le renouvellement du milieu consiste à remplacer le milieu biologique dans lequel sont suspendues les cellules par un autre milieu biologique, dit milieu biologique de renouvellement ou milieu de renouvellement.

Ce milieu de renouvellement peut avoir la même composition que le milieu biologique d'origine ou une composition différente. Par exemple, le premier milieu biologique est supplémenté en sérum, et le milieu de renouvellement est exempt de sérum.

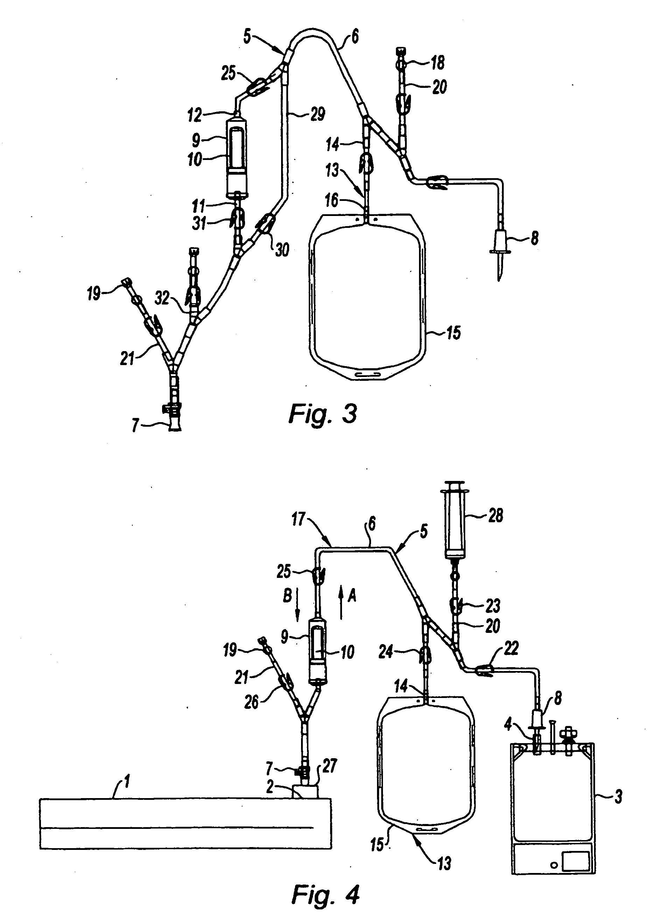

Selon une réalisation représentée sur la figure 4, le milieu biologique de renouvellement est conditionné dans un container 3 comprenant au moins un orifice d'entrée/sortie 4, notamment une poche ou un flacon. Ce container représente une source de milieu de renouvellement.

Le container est avantageusement biocompatible et stérilisable.

Le procédé selon l'invention prévoit de mettre en communication la suspension de cellules avec une source de milieu biologique de renouvellement par l'intermédiaire d'une voie de communication, ladite voie comprenant un moyen de rétention des cellules, et entre le moyen de rétention et la source, un moyen d'évacuation du milieu biologique.

Cette étape de mise en communication est réalisée notamment au moyen d'un système de transfert 5 tel que représenté sur les figures 2 ou 3.

Selon la figure 2, la voie de communication entre les cellules en suspension et le milieu de renouvellement comprend une première tubulure 6 munie à chacune de ses extrémités respectivement d'un moyen de mise en communication 7 avec un récipient 1 contenant la suspension et d'un moyen de mise en communication 8 avec un container contenant le milieu biologique de renouvellement.

Un moyen de rétention 9 est connecté à la première tubulure 6, ledit moyen de rétention étant capable de séparer les cellules du milieu biologique, c'est-à-dire capable de laisser passer le milieu biologique tout en retenant les cellules.

Après passage dans le moyen de rétention, la composition du milieu biologique reste substantiellement inchangée.

Le moyen de rétention 9 est par exemple un dispositif de filtration comprenant un filtre 10 ayant une porosité suffisamment petite pour empêcher le passage des cellules au travers du filtre et suffisamment grande pour laisser passer le milieu biologique.

Le filtre 10 se présente notamment sous la forme d'un grillage ou d'un canevas. La porosité du filtre varie en fonction de la taille des cellules à filtrer. Par exemple, pour des cellules de taille moyenne de 50 µm, une porosité de 40 µm est adaptée.

Les cellules ayant une taille pouvant être d'environ 10 µm, la porosité de filtre est par exemple comprise entre 5 et 60 µm.

De façon plus précise, le dispositif de filtration comprend une enveloppe extérieur définissant un volume intérieur. Le filtre est disposé dans le volume intérieur de sorte à définir un compartiment d'entrée et un compartiment de sortie. Le dispositif de filtration comprend un orifice d'entrée 11 en communication fluidique avec le compartiment d'entrée et un orifice de sortie 12 en communication fluidique avec le compartiment de sortie.

Par définition, l'orifice d'entrée 11 du dispositif de filtration est l'orifice le plus proche du récipient de culture 1.

Le filtre 10 est par exemple un filtre tubulaire réalisé en polypropylène. Le matériau du filtre est choisi en fonction de ses propriétés physico-chimiques pour éviter le colmatage du filtre par les cellules et favoriser la récupération des cellules. En effet, il faut éviter que les cellules adhèrent au filtre.

Selon la figure 4, le moyen de rétention 9 est placé sur la première tubulure 6 définissant la voie de communication entre la suspension contenue dans un récipient 1 et la source de milieu de renouvellement formé d'un container 3 contenant le milieu de renouvellement.

Des moyens de mise en communication 7,8 avec respectivement le récipient 1 contenant la suspension et le container 3 contenant le milieu de renouvellement sont arrangés aux extrémités de la première tubulure 6. Ces moyens de mise en communications sont par exemple des connecteurs, tels que des Luers, perforateurs, raccords, connecteurs stériles, etc.

En variante, la mise en communication avec le récipient 1 et/ou le container 3 contenant le milieu est réalisée par connexion stérile. Une telle connexion est par exemple décrite dans les documents EP-044 204, EP-0 134 630 et EP-0 208 004.

Notamment, l'une des extrémités de la première tubulure 6 comprend un raccord de type mâle pour connecter le récipient et l'autre extrémité comprend un perforateur pour connecter le container 3 contenant le milieu, dit container de milieu.

Selon l'invention, la voie de communication entre la source de milieu de renouvellement et la suspension comprend également un moyen d'évacuation 13 du milieu biologique.

Selon la figure 2, le moyen d'évacuation comprend une deuxième tubulure 14 connectée à la première tubulure 6, et un container de recueil 15 du milieu biologique qui est branché sur ladite deuxième tubulure 14.

Le container de recueil 15 est souple ou rigide. Il comprend au moins un orifice d'entrée/sortie 16. Notamment, le container 15 est sous forme d'une poche souple et stérilisable.

Avantageusement, le container 15 est intégré au moment de la fabrication au système de transfert par l'intermédiaire de son orifice 16 d'entrée/sortie.

Ce container de recueil 15 est destiné à recevoir le milieu biologique contenu initialement dans le récipient 1 de culture.

Le container de recueil 15 est réalisé en matériau polymère thermoplastique tel que le polychlorure de vinyle, l'alcool polyvinylique, le polyéthylène téréphtalate, le polystyrène ou l'éthylène - acétate de vinyle.

Pour réaliser le renouvellement selon l'invention, le procédé prévoit, après l'étape de mise en communication, d'évacuer dans le moyen d'évacuation 13 le milieu biologique de la suspension par passage au travers du moyen de rétention 9 dans un premier sens, indiqué par la flèche A, sur la figure 4.

Selon la figure 4, cette étape est notamment réalisée en positionnant le récipient 1 de culture au dessus du container de recueil 15 de sorte à faire s'écouler par gravité le milieu biologique du récipient 1 dans le container 15 de recueil par passage dans le dispositif de filtration. Les cellules en suspension dans le milieu biologique sont retenues dans le filtre 10, seul le milieu biologique pouvant traverser le filtre 10.

Avantageusement, cette manipulation est réalisée avec précaution, c'est-à-dire, en déplaçant lentement le récipient 1 de sorte à ce que les cellules restent au maximum au fond du récipient. Ainsi, lors de l'évacuation, un minimum de cellules sort du récipient 1, limitant le risque de colmatage du filtre 10 du moyen de rétention 9.

L'évacuation du milieu biologique est totale ou partielle. Notamment, lorsque le milieu biologique comprend des produits d'intérêts, comme par exemple des protéines thérapeutiques, l'évacuation est totale.

Une évacuation partielle permet de limiter le nombre de cellules qui traversent le filtre 10 et donc un éventuel colmatage.

Selon l'invention, le procédé prévoit ensuite de transférer le milieu de renouvellement au travers du moyen de rétention 9 dans un deuxième sens, opposé au premier sens et indiqué par la flèche B sur la figure 4, pour mettre en suspension les cellules avec le milieu de renouvellement dans le récipient 1.

Ainsi, les cellules retenues précédemment dans le filtre 10 sont renvoyées dans le récipient 1 de culture. A la fin de cette étape, le récipient de culture contient les cellules en suspension dans le milieu de renouvellement.

Cette étape est notamment réalisée en surélevant le container de milieu 3 par rapport au récipient 1 de culture et en laissant couler par gravité le milieu de renouvellement dans le récipient 1 de culture par passage au travers du dispositif de filtration.

L'absence de centrifugation pendant ce renouvellement de milieu permet de manipuler des cellules fragiles.

De façon avantageuse, les étapes de procédé d'évacuation et de transfert sont réalisées en système clos, c'est-à-dire sans contact avec l'air ambiant. Ainsi, il n'est plus nécessaire de travailler sous hotte à flux laminaire, ce qui facilite les manipulations d'évacuation et de transfert ou autres manipulation d'observation des cellules, comptage, etc.

Le système clos est obtenu après que tous les éléments tels que le récipient de culture 1, le système de transfert 5 et le container 3 contenant le milieu de renouvellement sont reliés entre eux de sorte à former un ensemble de culture cellulaire 17, illustré sur la figure 4.

Selon le deuxième aspect de l'invention et en relation avec les figures 2 et 4, le système de transfert 5 comprend une première tubulure 6 dont la première extrémité est pourvue d'un moyen de mise en communication 7 avec le récipient 1 contenant la suspension et la deuxième extrémité est pourvue d'un moyen de mise en communication 8 avec le container 3 contenant le milieu biologique de renouvellement, un moyen de rétention 9 étant connecté sur ladite tubulure 6, une deuxième tubulure 14 étant connectée à la première tubulure 6 entre le moyen de rétention 9 et la deuxième extrémité, un container de recueil 15 étant branché sur la deuxième tubulure 14.

Comme représenté sur la figure 2, le système de transfert 5 comprend en outre au moins un autre moyen 18,19 de mise en communication avec une source de fluide pour permettre l'introduction ou le retrait du fluide dans le récipient de culture ou le container de milieu.

Par exemple, le système de transfert comprend une troisième tubulure 20 connectée à la première tubulure 6, entre le moyen 8 de mise en communication du container de milieu et la connexion avec la deuxième tubulure 14. Cette troisième tubulure 20 est pourvue d'un connecteur de type Luer, pour permettre par exemple l'introduction d'additifs dans le milieu de renouvellement.

De même, une quatrième tubulure 21 est connectée à la première tubulure 6 entre le moyen de rétention 9 et le moyen 7 de mise en communication avec le récipient 1. La quatrième tubulure 21 est pourvue d'un connecteur de type Luer pour permettre la récupération des cellules comme expliquer plus bas.

Avantageusement, les tubulures 6, 14, 20, 21 du système de transfert 5 sont munies de moyens sélectifs d'ouverture et de fermeture de la communication fluidique à travers des tubulures. Par exemples, toutes les tubulures sont munies d'au moins un clamp 22-26.

Les connexions entre les différentes tubulures sont réalisées via un connecteur 3 voies en forme de T ou Y.

Selon le troisième aspect de l'invention et comme représenté sur la figure 4, l'ensemble de culture cellulaire 17 formant le système clos comprend ledit système de transfert 5, et comprend:
- un récipient 1 de culture dans lequel sont placés des cellules et un milieu biologique comprenant au moins une voie d'accès 2, ladite voie étant connectée à l'une des extrémités de la première tubulure 6, et
- un container 3 dans lequel est placé le milieu de renouvellement et comprenant au moins un orifice 4 d'entrée/sortie, ledit orifice étant connecté à l'autre extrémité de la première tubulure 6.

En relation avec la figure 4, on décrit un exemple de procédé de renouvellement utilisant l'ensemble de culture tel que représenté.

Tous les clamps 22 à 26 sont initialement fermés. On procède à la connexion du système de transfert 5 et du récipient 1.

Le bouchon amovible du récipient est retiré et remplacé par un raccord 27 (figure 1) de type femelle. Le raccord comprend notamment un moyen de verrouillage de type clapet pour maintenir fixement le raccord mâle du système de transfert. Les raccords mâle et femelle sont connectés alors de façon étanche.

Le container 3 contenant le milieu de renouvellement est connecté de façon étanche au système de transfert 5, via le perforateur.

Une fois le récipient 1 et le container 3 connectés avec le système de transfert 5, le procédé est réalisé en système clos. Ainsi, le risque de contamination de la suspension cellulaire est minimisé et la manipulation de changement de milieu simplifiée et sécurisée.

Selon la figure 4, une seringue 28 est connectée au Luer placé sur la troisième tubulure 20, la seringue comprenant des additifs pour le milieu de renouvellement. Les deux clamps 22,23 disposés entre le Luer et le container de milieu sont ouverts, et les additifs injectés dans le container 3 de milieu. Les deux clamps 22,23 sont alors refermés et la seringue 28 est laissée connectée au système de transfert 5 pour maintenir le système clos.

Pour réaliser l'évacuation du milieu biologique du récipient, les deux clamps 24,25 situés entre le moyen de rétention 9 et le container de recueil 15 sont ouverts. Le milieu biologique est évacué du récipient 1 de culture dans le container 15 de recueil par passage au travers du moyen de rétention 9, dans le sens indiqué par la flèche A. Une fois l'évacuation terminée, le clamp 24 sur la deuxième tubulure est fermé.

Le transfert du milieu de renouvellement est réalisé après ouverture des clamps 22,25 de la première tubulure 6. Le milieu de renouvellement est transféré par gravité vers le récipient 1 de culture en surélevant le container 3 de milieu. Le milieu de renouvellement doit rincer le filtre 10 dans le sens indiqué par la flèche B pour que toutes les cellules se retrouvent dans le récipient de culture.

Les clamps 22,25 de la première tubulure 6 sont ensuite refermés. Une fois le renouvellement achevé, le système de transfert 5 est dissocié du récipient 1 de culture par déconnexion des raccords et le bouchon amovible replacé sur la voie d'accès 2 du récipient de culture 1.

A la fin de la culture des cellules, les cellules peuvent être récupérées directement dans une seringue ou une poche via le Luer de la quatrième tubulure 21 en ouvrant le clamp 26 placé sur la quatrième tubulure, sans passer par le filtre.

A ce moment, il est avantageux d'évacuer un maximum de milieu biologique dans le container de recueil 15 comme expliqué ci-dessus.

Cette récupération évite encore une fois une centrifugation qui soumettrait les cellules à un stress supplémentaire.

La figure 3 représente une variante du système de transfert selon l'invention.

En plus des éléments du système de transfert 5 de la figure 2, ce système comprend une cinquième tubulure 29 connectée sur la première tubulure de sorte à définir une voie de dérivation contournant le moyen de rétention 9.

Selon la figure 2, une des extrémités de la cinquième tubulure 29 est connectée entre le moyen 7 de mise en communication avec le récipient de culture et l'entrée 11 du dispositif de filtration, et l'autre extrémité entre la connexion avec la deuxième tubulure 14 et la sortie 12 du dispositif de filtration.

Cette voie de dérivation est utilisée pour le renouvellement d'un milieu biologique dans le cas de culture de cellules adhérentes, c'est-à-dire attachées au fond du récipient de culture. L'évacuation et le transfert des milieux est réalisée de façon similaire aux étapes décrites ci-dessus, en utilisant la voie de dérivation pour éviter le passage au travers du filtre.

Des moyens sélectifs d'ouverture et de fermeture de la communication fluidique à travers les tubulures, tels que des clamps 25, 30, 31 placés de part et d'autre du moyen de rétention 9 et que la cinquième tubulure 26, permettent d'obliger le passage dans la voie de communication contenant le moyen de rétention ou dans la voie de dérivation.

Selon la figure 3, le système de transfert 5 comprend en outre une sixième tubulure 32 connectée sur la première tubulure 6 entre le moyen 7 de mise en communication du récipient 1 et la connexion avec la cinquième tubulure 29. La sixième tubulure 32 comprend un connecteur de type Luer pour connecter une seringue ou une poche. Cette connexion est notamment utilisée pour injecter dans le récipient de culture des substances utiles pour la culture de cellules ou pour les dissocier et les détacher du récipient en vue de leur récupération.

Ainsi, l'utilisateur est libre d'utiliser ce système de transfert 5 pour la culture de cellules adhérentes ou non adhérentes et peut réaliser dans les deux cas, après association avec un récipient de culture 1 et un container 3 contenant un milieu de renouvellement, le renouvellement du milieu en système clos.

## Revendications

1. Procédé de renouvellement d'un milieu biologique dans lequel des cellules sont en suspension, ledit procédé prévoyant de :
- mettre en communication la suspension avec une source de milieu biologique de renouvellement par l'intermédiaire d'une voie de communication, ladite voie comprenant un moyen de rétention (9) des cellules et, entre le moyen de rétention (9) et la source, un moyen d'évacuation (13) du milieu biologique ;
- évacuer dans le moyen d'évacuation (13) le milieu biologique de la suspension par passage au travers du moyen de rétention (9) dans un premier sens ; puis
- transférer le milieu de renouvellement au travers du moyen de rétention (9) dans un deuxième sens, pour mettre en suspension les cellules avec le milieu de renouvellement, ledit deuxième sens étant prévu pour renvoyer dans la suspension les éventuelles cellules retenues dans le dispositif de rétention (9).

2. Procédé selon la revendication 1, **caractérisé en ce que** les cellules sont des cellules non-adhérentes.

3. Procédé selon la revendication 1 ou 2, **caractérisé en ce que** le moyen de rétention (9) comprend un filtre (10) de porosité comprise entre 5 et 60 µm.

4. Procédé selon l'une quelconque des revendications 1 à 3, **caractérisé en ce qu'**au moins un milieu biologique est un milieu nutritif.

5. Procédé selon l'une quelconque des revendications 1 à 4, **caractérisé en ce que** les étapes du procédé sont réalisées en système clos.

6. Procédé selon la revendication 5, **caractérisé en ce que** la voie de communication comprend une première tubulure (6) munie à chacune de ses extrémités respectivement d'un moyen (7) de mise en communication avec un récipient (1) contenant la suspension et d'un moyen (8) de mise en communication avec un container (3) contenant le milieu biologique de renouvellement.

7. Procédé selon la revendication 6, **caractérisé en ce que** le moyen d'évacuation (13) comprend une deuxième tubulure (14) connectée à la première tubulure (6), et un container de recueil (15) du milieu biologique qui est branché sur la deuxième tubulure (14).

8. Système de transfert (5) pour la mise en oeuvre du procédé selon la revendication 7, ledit système comprenant une première tubulure (6) dont l'une première extrémité est pourvue d'un moyen (7) de mise en communication avec le récipient (1) contenant la suspension et la deuxième extrémité est pourvue d'un moyen (8) de mise en communication avec le container (3) contenant le milieu biologique de renouvellement, un moyen de rétention (9) étant connecté sur ladite tubulure (6), une deuxième tubulure (14) étant connectée à la première tubulure (6) entre le moyen de rétention (9) et la deuxième extrémité, un container de recueil (15) étant branché sur la deuxième tubulure (14).

9. Système de transfert selon la revendication 8, **caractérisé en ce que** les tubulures (6, 14, 20, 21) sont munies de moyens sélectifs d'ouverture et de fermeture de la communication fluidique à travers lesdites tubulures.

10. Système selon la revendication 8 ou 9, **caractérisé en ce que** le moyen de rétention (9) comprend un filtre (10) de porosité comprise entre 5 et 60 µm.

11. Système selon l'une quelconque des revendications 8 à 10**, caractérisé en ce qu'**il comprend en outre au moins un autre moyen (18,19) de mise en communication avec une source de fluide pour permettre l'introduction ou le retrait du fluide dans le récipient de culture ou le container de milieu.

12. Système selon l'une quelconque des revendications 8 à 11, **caractérisé en ce qu'**il comprend en outre une tubulure (29) connectée sur la première tubulure (6) de sorte à définir une voie de dérivation contournant le moyen de rétention (9).

13. Ensemble de culture cellulaire (17) comprenant un système de transfert (5) selon l'une quelconque des revendications 8 à 12, comprenant :
- un récipient (1) de culture dans lequel sont placés des cellules et un premier milieu biologique comprenant au moins une voie d'accès (2), ladite voie étant connectée à l'une des extrémités de la première tubulure (6) ;
- un container (3) dans lequel est placé un milieu biologique de renouvellement et comprenant au moins un orifice d'entrée/sortie (4), ledit orifice étant connecté à l'autre extrémité de la première tubulure (6).

14. Ensemble selon la revendication 13, **caractérisé en ce que** le récipient (1) est un récipient rigide, adapté à la culture des cellules non adhérentes.

## Claims

1. Process for renewing a biological medium in which cells are suspended; said process involves:
- placing the suspension in communication with a biological medium renewal source by means of a communication channel, in which said channel includes means for holding (9) cells, and, between the holding means (9) and the source, means for discharging (13) the biological medium;
- discharging the biological medium of the suspension into the discharge means (13) by passing through the holding means (9) in a first direction; then
- transferring the renewal medium by means of the holding means (9) in a second direction, so as to place the cells in suspension with the renewal medium, in which said second direction is intended to return any cells held in the holding device (9) to the suspension.

2. Process according to claim 1, **characterized in that** the cells are non-adherent cells.

3. Process according to claim 1 or 2, **characterized in that** the holding means (9) include a filter (10) with a porosity of between 5 and 60 µm.

4. Process according to any one of claims 1 to 3, **characterized in that** at least one biological medium is a nutrient medium.

5. Process according to any one of claims 1 to 4, **characterized in that** the steps of the process are performed in a closed system.

6. Process according to claim 5, **characterized in that** the communication channel includes a first tubing (6) equipped at each of its ends with means (7) providing communication with a container (1) containing the suspension and means (8) providing communication with a container (3) containing the biological renewal medium.

7. Process according to claim 6, **characterized in that** the discharge means (13) include a second tubing (14) connected to the first tubing (6), and a container (15) for collecting the biological medium, which is connected to the second tubing (14).

8. Transfer system (5) for implementing the process according to claim 7, in which said system includes a first tubing (6) of which a first end is equipped with means (7) providing communication with the container (1) containing the suspension and the second end is equipped with means (8) providing communication with the container (3) containing the biological renewal medium, in which holding means (9) are connected to said tubing (6), a second tubing (14) being connected to the first tubing (6) between the holding means (9) and the second end, and a collection container (15) is connected to the second tubing (14).

9. Transfer system according to claim 8, **characterized in that** the tubings (6, 14, 20, 21) are equipped with selective means for opening and closing the fluidic communication through said tubings.

10. System according to claim 8 or 9, **characterized in that** the holding means (9) include a filter (10) with a porosity between 5 and 60 µm.

11. System according to any one of claims 8 to 10, **characterized in that** it also includes at least one other means (18, 19) providing communication with a fluid source so as to enable the fluid to be introduced or removed from the culture container or the medium container.

12. System according to any one of claims 8 to 11, **characterized in that** it also includes a tubing (29) connected to the first tubing (6) so as to define a bypass channel bypassing the holding means (9).

13. Cell culture assembly (17) including a transfer system (5) according to any one of claims 8 to 12, including:
- a culture container (1) in which cells are placed and a first biological medium including at least one access channel (2), in which said channel is connected to one of the ends of the first tubing (6);
- a container (3) in which a biological renewal medium is placed, and including at least one inlet/outlet orifice (4), in which said orifice is connected to the other end of the first tubing (6).

14. Assembly according to claim 13, **characterized in that** the container (1) is a rigid container, suitable for the culture of non-adherent cells.

## Patentansprüche

1. Verfahren zur Erneuerung eines biologischen Mediums, in dem sich Zellen in Suspension befinden, wobei das Verfahren Folgendes umfasst:
- Verbinden der Suspension mit einer Quelle eines biologischen Mediums zur Erneuerung mithilfe einer Verbindungsleitung, wobei die Leitung ein Rückhaltemittel (9) für die Zellen und zwischen dem Rückhaltemittel (9) und der Quelle ein Ableitungsmittel (13) für das biologische Medium umfasst;
- Ableiten des biologischen Mediums der Suspension im Ableitungsmittel (13) nach Durchlauf durch das Rückhaltemittel (9) in einer ersten Richtung; und
- Einleiten des Mediums zur Erneuerung durch das Rückhaltemittel (9) in einer zweiten Richtung, um die Zellen mit dem Medium zur Erneuerung in Suspension zu bringen, wobei die zweite Richtung vorgesehen ist, um eventuell im Rückhaltemittel (9) zurückgehaltene Zellen in die Suspension zurückzuführen.

2. Verfahren nach Anspruch 1, **dadurch gekennzeichnet, dass** die Zellen nicht adhärente Zellen sind.

3. Verfahren nach Anspruch 1 oder 2, **dadurch gekennzeichnet, dass** das Rückhaltemittel (9) einen Filter (10) mit einer Porosität zwischen 5 und 60 µm umfasst.

4. Verfahren nach einem der Ansprüche 1 bis 3, **dadurch gekennzeichnet, dass** mindestens ein biologisches Medium ein Nährmedium ist.

5. Verfahren nach einem der Ansprüche 1 bis 4, **dadurch gekennzeichnet, dass** die Verfahrensschritte als geschlossenes System ausgeführt werden.

6. Verfahren nach Anspruch 5, **dadurch gekennzeichnet, dass** die Verbindungsleitung eine erste Rohrverzweigung (6) umfasst, die an jedem ihrer Enden mit jeweils einem Mittel (7) zur Verbindung mit einem Gefäß (1), das die Suspension enthält, und einem Mittel (8) zur Verbindung mit einem Behälter (3), der das biologische Medium zur Erneuerung enthält, ausgestattet ist.

7. Verfahren nach Anspruch 6, **dadurch gekennzeichnet, dass** das Ableitungsmittel (13) eine zweite Rohrverzweigung (14), die an die erste Rohrverzweigung (6) angeschlossen ist, und einen Sammelbehälter (15) für das biologische Medium umfasst, der an die zweite Rohrverzweigung (14) angeschlossen ist.

8. Transfersystem (5) für die Umsetzung des Verfahrens nach Anspruch 7, wobei das System eine erste Rohrverzweigung (6), von der ein erstes Ende mit einem Mittel (7) zur Verbindung mit einem Gefäß (1), das die Suspension enthält, ausgestattet ist und das zweite Ende mit einem Mittel (8) zur Verbindung mit einem Behälter (3), der das biologische Medium zur Erneuerung enthält, ausgestattet ist, ein Rückhaltemittel (9), das an die Rohrverzweigung (6) angeschlossen ist, eine zweite Rohrverzweigung (14), die an die erste Rohrverzweigung (6) zwischen dem Rückhaltemittel (9) und dem zweiten Ende angeschlossen ist, und einen Sammelbehälter (15) umfasst, der an die zweite Rohrverzweigung (14) angeschlossen ist.

9. Transfersystem nach Anspruch 8**, dadurch gekennzeichnet, dass** die Rohrverzweigungen (6, 14, 20, 21) mit selektiven Mitteln zum Öffnen und Schließen der fluidischen Verbindung durch die Rohrverzweigungen ausgestattet sind.

10. System nach Anspruch 8 oder 9, **dadurch gekennzeichnet, dass** das Rückhaltemittel (9) einen Filter (10) mit einer Porosität zwischen 5 und 60 µm umfasst.

11. System nach einem der Ansprüche 8 bis 10, **dadurch gekennzeichnet, dass** es ferner mindestens ein weiteres Mittel (18, 19) für die Verbindung mit einer Fluidquelle umfasst, um das Ein- oder Ableiten des Fluids im Gefäß mit der Kultur oder im Behälter mit dem Medium zu ermöglichen.

12. System nach einem der Ansprüche 8 bis 11, **dadurch gekennzeichnet, dass** es ferner eine Rohrverzweigung (29) umfasst, die an die erste Rohrverzweigung (6) so angeschlossen ist, dass eine Umleitungsstrecke definiert wird, die das Rückhaltemittel (9) umgeht.

13. Zellkultureinheit (17), umfassend ein Transfersystem (5) nach einem der Ansprüche 8 bis 12, Folgendes umfassend:
- ein Gefäß (1) mit einer Kultur, in dem Zellen und ein erstes biologisches Medium angeordnet sind, umfassend mindestens eine Zugangsleitung (2), wobei die Leitung an eines der Enden der ersten Rohrverzweigung (6) angeschlossen ist;
- einen Behälter (3), in dem ein biologisches Medium zur Erneuerung angeordnet ist, und der mindestens eine Eintritts-/Austrittsöffnung (4) umfasst, wobei die Öffnung an das andere Ende der ersten Rohrverzweigung (6) angeschlossen ist.

14. Einheit nach Anspruch 13, **dadurch gekennzeichnet, dass** das Gefäß (1) ein starres Gefäß ist, das an die Kultur nicht adhärenter Zellen angepasst ist.
